# EUROPEAN PATENT SPECIFICATION

(11) **EP 3 616 723 B1**
(45) Date of publication and mention of the grant of the patent: **07.08.2024**
(21) Application number: 19749168.1
(22) Date of filing: 22.03.2019
(51) Int. Cl.: A61K 31/517, A61K 31/455, A61P 27/10, A61K 36/236, A61K 36/537, A61K 36/488

(54) **USE OF NICOTINIC ACID OR PRAZOSIN FOR TREATING MYOPIA**
VERWENDUNG VON NIKOTINSÄURE ODER PRAZOSIN ZUR BEHANDLUNG VON MYOPIE
UTILISATION D'ACIDE NICOTINIQUE OU DE PRAZOSINE POUR LE TRAITEMENT DE LA MYOPIE

(30) Priority: 18.07.2018 CN 201810787541
(43) Date of publication of application: 04.03.2020
(73) Proprietor: Wenzhou Medical University, Wenzhou, Zhejiang 325000 (CN)
(72) Inventor: ZHOU, Xiangtian, Wenzhou Zhejiang 325000 (CN); ZHANG, Sen, Wenzhou Zhejiang 325000 (CN); QU, Jia, Wenzhou Zhejiang 325000 (CN); ZHANG, Guoyun, Wenzhou Zhejiang 325000 (CN); ZHAO, Fei, Wenzhou Zhejiang 325000 (CN); ZHOU, Xuan, Wenzhou Zhejiang 325000 (CN)
(74) Representative: Viering, Jentschura & Partner mbB Patent- und Rechtsanwälte
(86) International application number: PCT/CN2019/079184
(87) International publication number: WO 2020/015377

(56) References cited:
- WO-A1-00/54773
- WO-A1-02/24191
- WO-A1-2016/171282
- CN-A- 1 128 656
- CN-A- 1 309 971
- CN-A- 1 500 494
- CN-A- 1 878 569
- CN-A- 102 552 091
- CN-A- 107 320 725
- WU HAO ET AL: "Scleral hypoxia is a target for myopia control", PROCEEDINGS OF THE NATIONAL ACADEMY OF SCIENCES, vol. 115, no. 30, 9 July 2018 (2018-07-09), XP055902520, ISSN: 0027-8424, DOI: 10.1073/pnas.1721443115

## Description

### TECHNICAL FIELD

The present invention relates to nicotinic acid or prazosin for use in treating axial myopia caused by scleral hypoxia by increasing the volume of choroidal blood flow.

### BACKGROUD

A choroid plays an important role in refractive development and myopia occurrence. It has been found through a large number of animal experiments that the choroidal thickness is thinned when the myopia is formed. Similar results are observed in human population, and the choroidal thickness of a hypermetropic human population is significantly thicker than that of each of emmetropic and myopic human populations. Our research team has found that, the thinning of the choroidal thickness occurs in addition to changing of an ocular anterior segment when human eyes are adjusted for looking the close, but the mechanism of this change is not well understood.

A sclera is a final effector for the occurrence and development of myopia, where after the myopia occurs, collagen fibers of the sclera become thinner and have a disordered arrangement, the scleral collagen is reduced, and the sclera is thinned. However, its regulatory mechanisms and trigger factors are still unclear. There are many types of cells in the sclera. In the past research, the whole scleral tissue is taken as a research object, such that the information derived from different cell sources is mixed and cannot accurately reveal cell-specific changes, and thus it is difficult to accurately explain the regulation mechanism of collagen synthesis in a scleral fibroblast. Our team has found that expression of a scleral hypoxia-inducible factor 1α (H IF-1α) is up-regulated when the myopia occurs, and returned back to normal when the myopia is recovered, suggesting that the scleral tissue is in an anoxic state when the myopia occurs.

Hypoxia of the scleral tissue may be a cause for extracellular matrix remodeling and myopia. The sclera belongs to a connective tissue, which itself has fewer blood vessels, and a large part of oxygen is derived from choroidal vessels. The choroid is mainly composed of a vascular network, so changing of its thickness is likely to result from regulation of choroidal blood flow. The function of the choroid is mainly supplying nutrients and oxygen to a retina and participating in regulation of scleral growth.

CN 1 500 494 relates to a composite preparation for the treatment of myopia, hyperopia, strabismus and amblyopia, characterized in that the composite preparation comprises zinc sulfate (10-25 wt.%), Vitamin B1 (25-30 wt.%), Vitamin B2 (5-10 wt.%), Vitamin B6 (10-15 wt.%), and Niacin (15-25 wt.%).

CN 1 878 569 relates to methods and compositions for the treatment of conditions including ocular, stress-associated, and neurodegenerative conditions in a mammal using a composition which directly or indirectly stimulates alpha 2 adrenoreceptor agonist activity with a minimum of sedation or other side effects.

CN 1 309 971 relates to an eye drop for treating myopia with said eye drop comprising Dibazol or Anisodamine, Inosine, vitamin B1, Adenosine Tripholsphate and vitamin B12. The water solvent of said eye drop is deionized water, 0.9% physiological saline or their mixture, into which ethanol or polyol in 2-18 % as stabilizer and preservative in 0.25-1 % may be added.

CN 1 128 656 relates to eyedrops is prepd. from anisodamine, sodium hyaluronate and antiseptics for the treatment of myopia of youngsters caused by unproperly using of the eyes and fatigue of vision.

WO 02/24191 relates to the use of NO synthase substrate and/or NO donor, or inducible NO synthase induce inhibitor and/or inducible NO synthase activity inhibitor for the preparation of drugs for treating or preventing myopia.

WO 00/54773 relates to compositions comprising at least one dopamine agonist in combination with at least one nitric oxide donor for treating and/or preventing sexual dysfunctions and/or enhancing sexual responses in patients. and for treating or preventing neurodegenerative diseases, mitochondrial diseases, spinal cord injury, central or psychostimulant addiction, senile dementia, circulatory disorders, cardiovascular disorders, hyperprolactinaemia or myopia.

WO 2016/171282 relates to an agent for preventing myopia and a myopia progression inhibitor. Specifically, it relates to an agent for preventing myopia or myopia progression inhibitor containing, as an active ingredient, a compound having a specific structural formula or a pharmacologically acceptable salt thereof.

### SUMMARY

### Technical problem

### Solution for the Problem

### Technical Solutions

In order to remedy the deficiencies of the prior art, an objective of the present invention is to provide compounds for use in treating axial myopia caused by scleral hypoxia by increasing the volume of choroidal blood flow.

The present invention is apparent from the appendant claim.

The drug that directly dilates a blood vessel or vascular smooth muscle is nicotinic acid.

The α-adrenergic receptor blocker is prazosin.

### BRIEF DESCRIPTION OF THE DRAWINGS

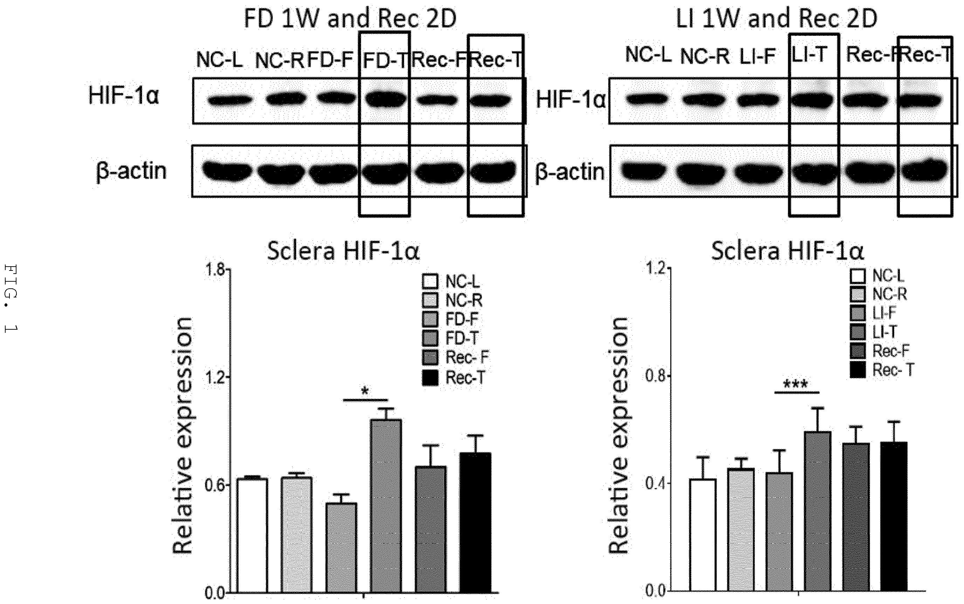

FIG. 1 shows the change of the scleral hypoxia-inducible factor 1α (HIF-1α) when myopia occurs; NC-L: Normal control left eye, NC-R: Normal control right eyed-F: Form-deprived fellow eye, FD-T: Form-deprived test eye, I-F: Minus-lens induced fellow eye, I-T: Minus-lens induced test eye, Rec-F: Recovered fellow eye, Rec-T: Recovered test eye.

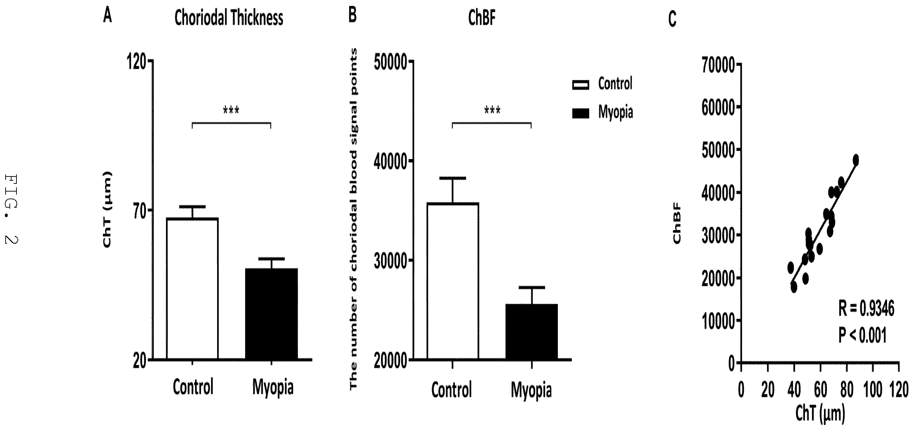

FIG. 2 is a diagram that shows the choroidal thickness and blood flow of spontaneous myopia and hyperopia; A: diagram of choroid thickness, B:diagram of choroidal blood flow, C: correlation between choroidal thickness and choroidal blood flow. Control: a normal group, Myopia: a spontaneous myopia group, Choroidal Thickness (ChT): choroidal thickness, Chorodal blood flow (ChBF): volume of choroidal blood flow.

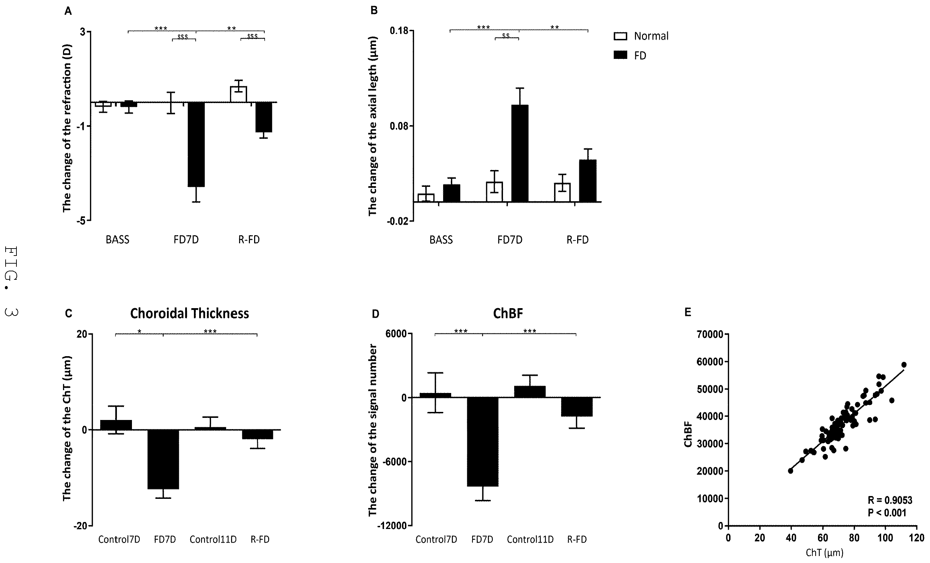

FIG. 3 is a diagram showing various parameters for 7 days of form deprivation and a recovery period: A: a diagram showing the difference of binocular refractions, B: a diagram showing the difference of binocular axial lengths, C: a diagram showing the difference of binocular choroidal thicknesses, D: a diagram showing the difference of binocular choroidal blood flows, E: Correlation between choroidal thickness and choroidal blood flow. Base: Before the test,Control7D: 7 days of a normal group,FD7D: 7 days of formal deprivation,Control11D: 11 days of a normal group, R-FD: 4 days after form deprivation, Choroidal Thickness (ChT): choroidal thickness, Chorodal blood flow (ChBF): choroidal blood flow.

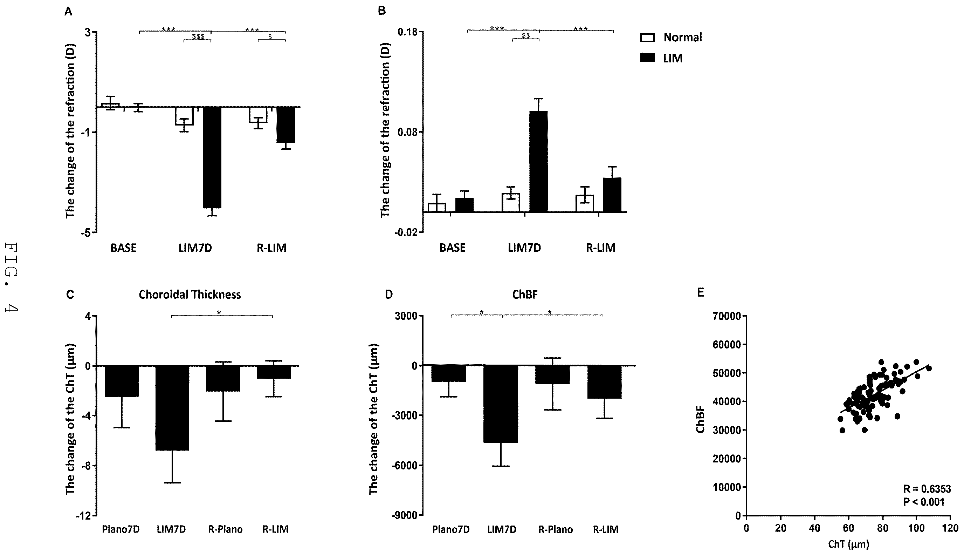

FIG. 4 is a diagram showing various parameters for 7 days of lens-induction and a recovery period: A: a diagram showing the difference of binocular refractions, B: a diagram showing the difference of binocular axial lengths, C: a diagram showing the difference of binocular choroidal thicknesses, D: a diagram showing the difference of binocular choroidal blood flows, E: Correlation between choroidal thickness and choroidal blood flow. Base: Before the test,Plano7D: 7 days of plano lens, LIM7D: 7 days of minus lens, R-Plano: 4 days with the plano lens removed, R-LIM: 4 days after the minus lens is removed, Choroidal Thickness (ChT): Choroidal thickness, Chorodal blood flow (ChBF): Choroidal blood flow.

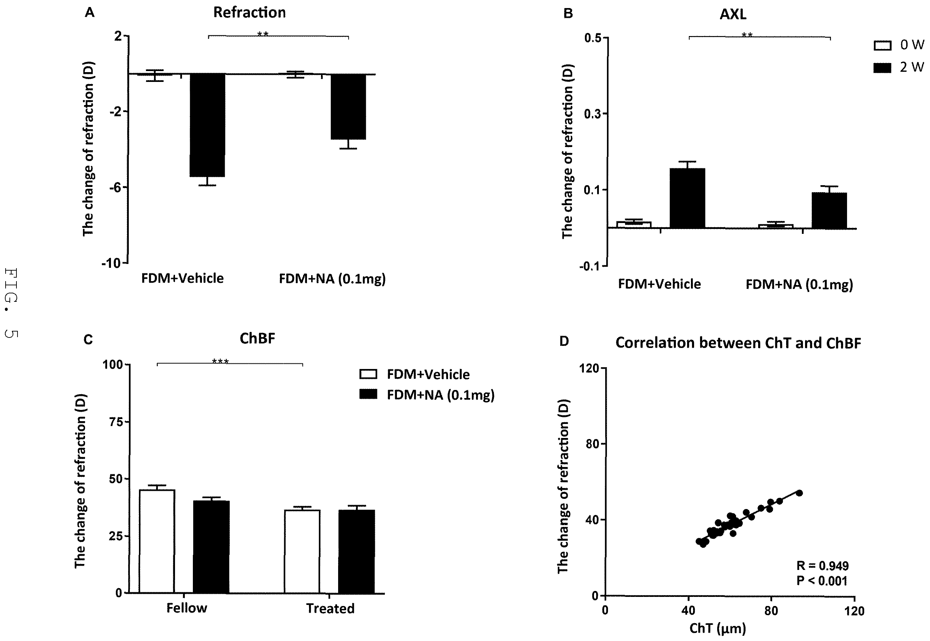

FIG. 5 is a schematic diagram showing the results for the effect of the drug (nicotinic acid) that directly dilates a blood vessel or vascular smooth muscle on form deprivation: Where A: a diagram showing the difference of binocular refractions, B: a diagram showing the difference of binocular axial lengths, C: a diagram showing binocular choroidal blood flows, D: Correlation between choroidal thickness and choroidal blood flow. Vehicle: Solvent, NA: nicotinic acid, ChT: choroidal thickness, Chorodal blood flow (ChBF): choroidal blood flow, ChBF Signal Points: signal points in the choroidal blood flow.

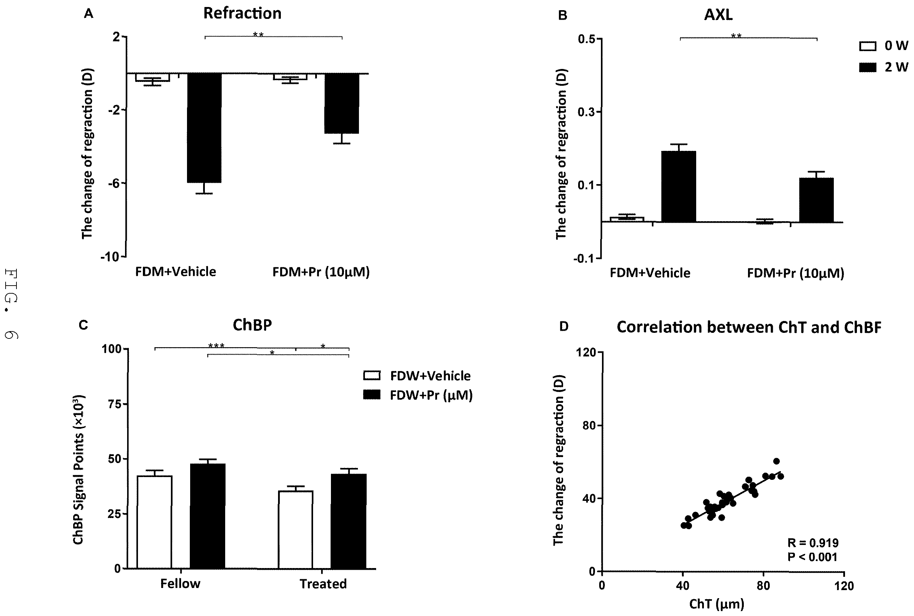

FIG. 6 is schematic diagram showing the results for the effect of the α-adrenergic receptor blocker (α-blocker: prazosin) on form deprivation: A: a diagram showing the difference of binocular refractions, B: a diagram showing the difference of binocular axial lengths, C: a diagram showing binocular choroidal blood flows, D: Correlation between choroidal thickness and choroidal blood flow. Vehicle: Solvent, Pr: prazosin, ChT: choroidal thickness, Chorodal blood flow (ChBF: choroidal blood flow, ChBF Signal Points: signal points in the choroidal blood flow.

### DESCRIPTION OF THE EMBODIMENTS

In order to explain the present invention more clearly, the specific embodiments are described below, and the specific embodiments do not limit the scope of the present invention.

Hypoxia of the scleral tissue may be a cause for extracellular matrix remodeling and myopia. The sclera belongs to a connective tissue, which itself has fewer blood vessels, and a large part of oxygen is derived from choroidal vessels. The choroid is mainly composed of a vascular network, so changing of its thickness is likely to result from regulation of choroidal blood flow. The function of the choroid is mainly supplying nutrients and oxygen to a retina and participating in regulation of scleral growth. Therefore, we have experimentally proved that the decrease of the volume of choroidal blood flow causes scleral hypoxia, thereby leading to myopia.

### Experimental Operations

(1) We conduct form deprivation or lens induction of 3week-aged guinea pigs, and find that expression of a scleral hypoxia-inducible factor 1α (HIF-1α) in a test eye is up-regulated when the myopia occurs, and returned back to normal when the myopia is recovered (FIG. 1), suggesting that the scleral tissue is in an anoxic state when the myopia occurs.
(2) Through detection of the choroids of 3-week-aged spontaneous myopia guinea pigs (refractive power: -5.94 ± 1.28 Diopter) and normal guinea pigs (refractive power: +3.36 ± 0.80 Diopter), we find that the choroidal thickness is significantly thinned and the blood flow is significantly reduced in the spontaneous myopia guinea pigs as compared with the normal guinea pigs.(normal group vs. spontaneous myopia group: choroid thickness: 67.65 ± 3.58 µm vs. 50.60 ± 3.14 µm, P < 0.01; choroidal blood flow: 35825.89 ± 2445.03 vs. 25633.22 ± 1665.09, P < 0.01). Also there is a high correlation between the choroidal thickness and the choroidal blood flow (R = 0.9346, p < 0.001), as shown in detail in FIG. 2.
(3) We conduct form deprivation for 7 days on the 3 week-aged guinea pigs (Form deprivation, FD) to produce an obvious myopia, and the myopia is significantly recovered 4 days after the deprivation factors are removed. (Base vs. FD 7D: 5.46 ± 0.65D vs. 1.00 ± 1.13D, p < 0.01; FD vs. R-FD: 1.00 ± 1.13D vs. 2.73 ± 0.63D, p < 0.05), and the eye axis has a corresponding response. The choroidal thickness is significantly thinned or the blood flow is significantly reduced on day FD7 and is statistically significant (normal group vs. FD group vs. R-FD: choroid thickness 69.8 ± 13.2 µm vs. 64.3 ± 11.3 µm vs. 77.0 ± 12.1 µm; choroidal blood flow:36548 ± 6368 vs. 32448 ± 5815 vs. 38706 ± 6356), and there is a high correlation between the choroidal thickness and the choroidal blood flow (R = 0.9053, p < 0.001), as shown in detail in FIG. 3.
(4) We conduct lens-induction for 7 days on the 3week-aged guinea pigs (Lens induced, LI) to produce an obvious myopia, and the myopia is significantly recovered 4 days after the induction factors are removed. (Base vs. LI 7D: 5.70 ± 0.25D vs. 1.11 ± 0.59D, p < 0.001; LI 7D vs. R-LIM: 1.11 ± 0.59D vs. 3.54 ± 0.43D, p < 0.001), and the eye axis has a corresponding response. The choroidal thickness is significantly thinned or the blood flow is significantly reduced on day LI7 and is statistically significant (normal group vs. LI group vs. R-LI: choroid thickness 73.1 ± 10.0 µm vs. 68.2 ± 7.9 µm vs. 74.6 ± 8.6 µm; choroidal blood flow:40887 ± 5116 vs. 38975 ± 3931 vs. 41965 ± 4012), and there is a high correlation between the choroidal thickness and the choroidal blood flow (R = 0.6353, p < 0.001), as shown in detail in FIG. 4.

We find that the decrease of the volume of choroidal blood flow causes scleral hypoxia, thereby leading to myopia. Through the above experiments, we believe that myopia can be effectively controlled by increasing blood flow and increasing oxygen supply through expansion of the choroid.

Therefore, in the present invention, the myopia caused by scleral hypoxia is treated by increasing the volume of choroidal blood flow.

In the present invention, by expanding the choroid via a drug, the oxygen supply to the sclera is increased, thereby increasing the volume of choroidal blood flow to achieve treatment of the axial myopia caused by scleral hypoxia.

The present invention can effectively control myopia by increasing blood flow and increasing oxygen supply through expansion of the choroid via the following drugs:
1. a drug that directly dilates a blood vessel or vascular smooth muscle: nicotinic acid;
2. a α-adrenergic receptor blocker (α- blocker): prazosin.

The mechanism is as follows:
1. The drug that expands the choroid is a drug that directly dilates a blood vessel or vascular smooth muscle.
   Principle: it has a direct relaxation effect on the vascular smooth muscle, reduces peripheral resistance and has a vasodilating effect.
2. the drug that expands the choroid is a α-adrenergic receptor blocker.
   Principle: an α-receptor blocker refers to a blocker that selectively binds to a α-adrenoceptor, does not agonize or attenuate agonization of adrenergic receptors, but can block the binding of corresponding neurotransmitters and drugs to the α-receptor to produce an anti-adrenergic effect. By effects of blocking the α1 receptor of the vascular smooth muscle and directly dilating the vascular smooth muscle, the blood vessels are dilated and the resistance is reduced.

One class is drugs that can compete for receptors with catecholamine to exert an effect of blocking the α-receptor, which work fast and act for a short duration due to the not very strong binding of them to the α-receptor, and are called short-acting α-receptor blockers. They are also known as competitive α-receptor blockers. Commonly used is phentolamine (Regitine). The other class is drugs that covalently bind to the α-receptor, have strong binding and features of having a strong receptor blocking action, long action time and the like, and are called long-acting class α-receptor blockers. They are also known as non-competitive α-receptor blockers, such as prazosin.

As shown in FIG. 5, we use a drug that directly dilate a blood vessel or a vascular smooth muscle: nicotinic acid (Niacin, NA), where on 2 weeks after form deprivation an obvious myopia is produced, and 0.1 mg of nicotinic acid can effectively suppress the form deprivation, FDM + Vehicle vs. FDM + NA (0.1 mg): -5.43 ± 1.69 vs. -3.43 ± 1.80 D, p < 0.01, FIG. 5A; the eye axis also has the corresponding effect of suppression, 0.17 ± 0.05 vs. 0.10 ± 0.06 mm , p < 0.01, FIG. 5B; on 2 weeks after form deprivation the blood flow in the test eye is significantly reduced and statistically significant, fellow eye vs. test eye:45.38 ± 6.22 vs. 36.62 ± 4.47 X10 3, p < 0.001, and after periocular injection of 0.1 mg nicotinic acid, there is no statistical difference between two eyes, FIG. 5C; and there is a high correlation between the choroidal blood flow and the choroidal thickness (R = 0.949, p < 0.001), as shown in detail in FIG. 5D .

As shown in FIG. 6, we use an α- adrenergic receptor blocker (α- blocker): prazosin (Prazosin, Pr) , where on 2 weeks after form deprivation, an obvious myopia is produced, and 10 µM of prazosin can effectively suppress the form deprivation, F DM + Vehicle vs. FDM + Pr (10 µM): - 5.98 ± 2.22 vs. -3.26 ± 2.22 D, p < 0.01, FIG. 6A; the eye axis also has the corresponding effect of suppression, 0.17 ± 0.05 vs. 0.10 ± 0.06 mm, p < 0.01 , FIG. 6B; on 2 weeks after form deprivation, the blood flow of the test eye is significantly reduced and statistically significant, fellow eye vs. test eye: 42.50 ± 8.05 vs. 35.79 ± 6.76 X10 3 , p < 0.001), and after periocular injection of 0.1 ml of 10 µM prazosin, the reduction of choroidal blood flow is significantly inhibited, FDM + Vehicle vs. FDM + Pr (10 µM): 35.79 ± 6.76 vs. 37.01 ± 6.26 X10 3, p < 0.05, FIG. 6C; and there is a high correlation between the choroidal blood flow and the choroidal thickness (R = 0.919, p < 0.001), FIG. 6D.

Therefore, we have demonstrated that, the myopia caused by scleral hypoxia can be treated by increasing the volume of choroidal blood flow through expansion of the choroid via a drug that dilates the blood vessels.

The above description is only a preferred embodiment of the present invention, and the claimed scope of the present invention is not limited to the above embodiments, and all the technical solutions under the inventive concept belong to the claimed scope of the present invention.

## Claims

1. Nicotinic acid or prazosin for use in treating axial myopia caused by scleral hypoxia by increasing the volume of choroidal blood flow.

## Patentansprüche

1. Nicotinsäure oder Prazosin zur Behandlung der durch sklerale Hypoxie verursachten axialen Myopie durch Erhöhung des choroidalen Blutflusses.

## Revendications

1. Acide nicotinique ou prazosine pour l'utilisation dans le traitement de la myopie axiale causée par l'hypoxie sclérale en augmentant le volume du flux sanguin choroïdien.
